# FASCICULE DE BREVET EUROPEEN

(11) **EP 1 510 190 B1**
(45) Date de publication et mention de la délivrance du brevet: **30.04.2008**
(21) Numéro de dépôt: 04356146.3
(22) Date de dépôt: 24.08.2004
(51) Int. Cl.: A61F 2/40

(54) **Composant glénoidien de prothèse d'épaule et prothèse totale d'épaule incorporant un tel composant**
Gelenkpfannenteil einer Schulterprothese und Schultertotalprothese mit einem solchen Teil
Glenoid component of a shoulder prosthesis and total shoulder prosthesis comprising such a component

(30) Priorité: 25.08.2003 FR 0310141
(43) Date de publication de la demande: 02.03.2005
(73) Titulaire: TORNIER, 38330 Saint-Ismier (FR)
(72) Inventeur: Tornier, Alain, 38330 Saint Ismier (FR)
(74) Mandataire: Myon, Gérard Jean-Pierre

(56) Documents cités:
- EP-A- 0 342 421
- EP-A- 0 903 127
- EP-A- 1 336 396
- WO-A-02/067821
- FR-A- 2 578 162
- FR-A- 2 652 498
- FR-A- 2 683 142
- FR-A- 2 704 747
- FR-A- 2 825 263
- US-A- 4 550 450
- US-A- 5 593 448
- US-A- 5 782 924
- US-A1- 2003 055 507
- US-B1- 6 406 495

## Description

La présente invention concerne un composant glénoïdien de prothèse d'épaule, ainsi qu'une prothèse totale d'épaule comprenant un tel composant.

Dans le domaine des prothèses d'épaule, il est courant d'utiliser un composant glénoïdien comprenant un corps concave en polyéthylène sur lequel vient s'appuyer une tête convexe métallique d'un composant huméral. C'est par exemple le cas dans US-A-5,593,448 et US-A-6,406,495. Il est en effet largement admis que c'est la tête humérale qui est la pièce la plus sollicitée mécaniquement durant le fonctionnement de la prothèse.

Cette solution largement employée présente cependant un certain nombre d'inconvénients liés à l'usure du corps glénoïdien en polyéthylène. En effet, on constate que ce corps en polyéthylène s'use assez rapidement et ce, d'autant plus que le contact entre le corps en polyéthylène et la tête humérale plus dure se forme sur une surface d'aire plus petite pour le corps en polyéthylène que pour la tête humérale. De plus, en cas de mauvais positionnement du corps glénoïdien par rapport à la tête humérale, l'usure est mal répartie. Sous l'effet d'efforts de basculement générés par la tête humérale mal positionnée, la prothèse risque alors d'être détériorée soit par descellement du composant glénoïdien, soit par luxation de l'articulation.

Pour contourner ces inconvénients, on a proposé par le passé soit d'augmenter l'épaisseur du corps en polyéthylène, ce qui cependant décale la surface d'articulation avec la tête humérale de sa position anatomique, soit d'interposer entre le corps en polyéthylène et la glène osseuse un insert métallique (on parle alors de composants glénoïdiens « metal-back »), comme décrit dans US-A-4,550,450. Cette dernière solution reste cependant coûteuse et aboutit couramment, surtout lorsque le positionnement entre la tête humérale et le corps glénoïdien n'est pas anatomiquement satisfaisant, a une désolidarisation du corps en polyéthylène par rapport à l'insert métallique.

Le but de la présente invention est de proposer un composant glénoïdien qui à la fois limite les risques de détérioration et de descellement de ce composant et qui coopère efficacement avec un composant prothétique huméral, tout en étant économique et facile à implanter.

A cet effet, l'invention a pour objet un composant glénoïdien de prothèse d'épaule, comprenant un corps métallique adapté, d'un côté, pour être immobilisé sur la glène d'une épaule, et portant, du côté opposé, une surface concave d'articulation adaptée pour coopérer avec un composant huméral de la prothèse, laquelle surface d'articulation se prolonge en périphérie par une surface convexe formant au moins en partie le chant du corps.

En utilisant le corps métallique du composant glénoïdien selon l'invention, on augmente de manière significative la rigidité de ce composant, sans pour autant nécessiter une épaisseur importante. L'usure de cette pièce métallique est quasiment nulle, réduisant ainsi fortement les risques de sa détérioration. En fonctionnement, la courbure de la surface d'articulation est prévue pour limiter les risques de luxation du composant huméral et la périphérie émoussée de cette surface évite de marquer, voire d'entailler le composant huméral. En outre, par rapport à des composants glénoïdiens « metal-back », le composant selon l'invention est plus économique et plus facile à implanter.

D'autres caractéristiques de ce composant glénoïdien, prises isolément ou selon toutes les combinaisons techniquement possibles, sont énoncées aux revendications dépendantes 2 à 12.

L'invention a également pour objet une prothèse totale d'épaule qui comprend un composant glénoïdien tel que défini ci-dessus et un composant huméral portant une surface convexe de coopération articulaire avec la surface concave du composant glénoïdien. Une telle prothèse présente l'avantage de faire porter l'usure sur le composant huméral, c'est-à-dire sur une plus grande surface portée par une matière plus épaisse. En outre, lorsque, au bout de plusieurs années, il est nécessaire de changer les pièces d'usure de la prothèse, seule la tête du composant huméral est à changer, rendant l'opération de reprise correspondante plus rapide et plus facile.

Suivant une caractéristique avantageuse de cette prothèse totale d'épaule, la surface convexe du composant huméral est portée par une pièce de ce composant constituée d'un matériau plus mou que le métal du corps du composant glénoïdien, notamment une matière plastique.

L'invention sera mieux comprise et d'autres avantages de celle-ci apparaîtront plus clairement à la lumière de la description qui va suivre, donnée uniquement à titre d'exemple et faite en référence aux dessins sur lesquels :
- la figure 1 est une représentation schématique de principe d'une prothèse conforme à l'invention, implantée sur un patient ;
- la figure 2 est une vue en élévation du composant glénoïdien de la prothèse de la figure 1 ;
- la figure 3 est une vue prise selon la flèche III indiquée sur la figure 2 ;
- la figure 4 est une vue en perspective d'une variante d'un composant glénoïdien selon l'invention ;
- la figure 5 est une coupe suivant le plan V du composant de la figure 4, implanté sur la glène d'un patient ;
- les figures 6 et 7 sont des vues en perspective, sous des angles de vue différents, d'une autre variante du composant glénoïdien selon l'invention ; et
- la figure 8 est une coupe suivant un plan sensiblement médian du composant des figures 6 et 7, implanté sur la glène d'un patient.

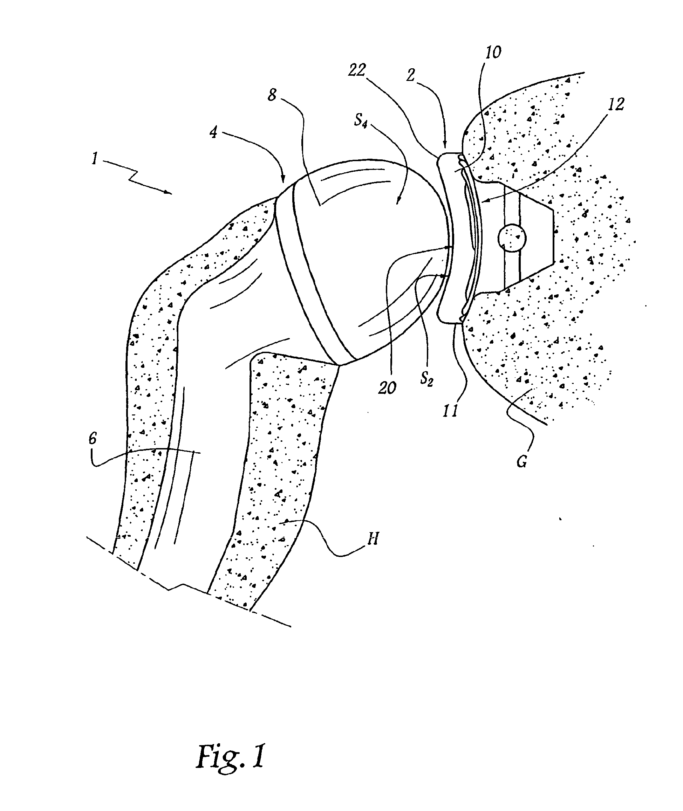

La prothèse 1 représentée sur la figure 1 comprend un premier composant 2 fixé sur la glène G d'une épaule, ainsi qu'un second composant 4 fixé dans l'humérus H correspondant.

Le composant 4 comprend, d'une part, une tige 6 destinée à être ancrée dans le canal médullaire de l'humérus H, et d'autre part, une tête hémisphérique 8 définissant une surface convexe S₄ en forme de tronçon de sphère.

La tige 6 est en métal, tandis que la tête 8 est, au moins dans sa partie périphérique, en matériau plus mou, notamment en polyéthylène, en particulier du polyéthylène haute densité (PEHD). La tête 8 est rapportée fixement sur la tige 6 par tout moyen connu, par exemple par emboîtement. La tête 8 est avantageusement adaptable sur des tiges humérales existantes.

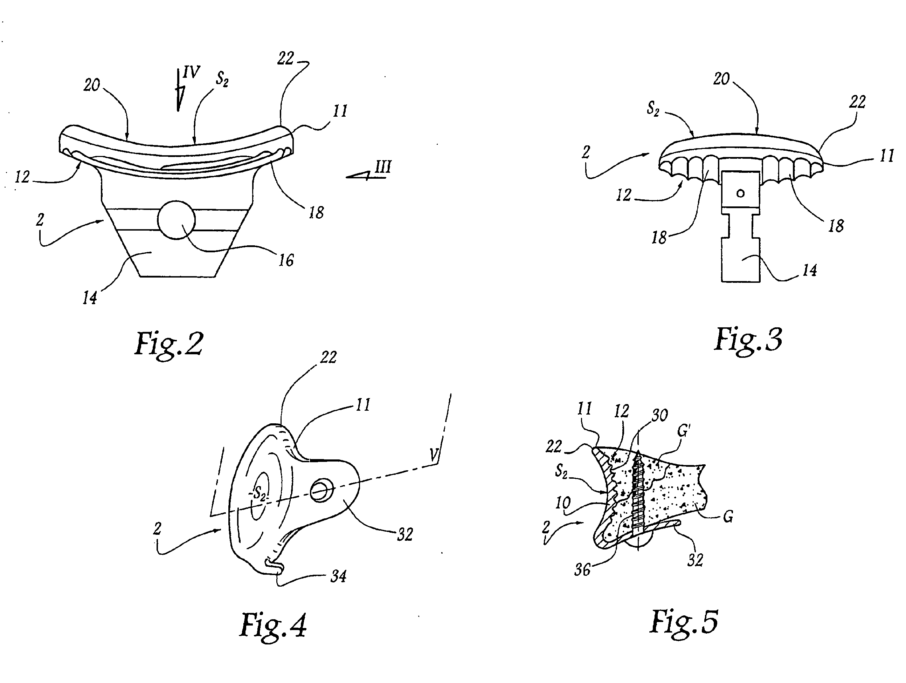

Par commodité, la suite de la description, notamment celle en rapport avec le composant glénoïdien 2, sera orientée en considérant que ce composant est dans sa position d'implantation vis-à-vis de la glène G. Plus précisément, en considérant le composant 2 représenté sur les figures 2 et 4, les termes « inférieur » et « supérieur » désignent respectivement les parties gauche et droite des figures 2 et 4, les termes « externe » et « interne » désignent les parties haute et basse de la figure 2, et les termes « antérieur » et « postérieur » désignent respectivement les parties haute et basse de la figure 4.

Le composant 2 des figures 2 à 4 comporte un corps métallique 10 d'un seul tenant, présentant suivant les directions antéro-postérieure et inférieure/supérieure des dimensions nettement supérieures à son épaisseur suivant la direction interne/externe. Le chant périphérique du corps 10 est référencé 11.

Le corps 10 présente ainsi une face interne 12 adaptée pour être immobilisée sur la glène G. Plus précisément, cette face 12 est munie d'une aile saillante 14 destinée à s'ancrer profondément dans la glène osseuse et dans laquelle est ménagé un trou 16 à l'intérieur duquel une vis d'immobilisation peut être insérée. Avantageusement, l'aile 14 est métallique et venue de matière avec le corps 10.

De part et d'autre de l'aile 14, la face 12 délimite des surfaces concaves 18 d'appui sur la cavité glénoidienne de l'omoplate du patient. Ces surfaces portent avantageusement des rainures qui s'étendent suivant la direction inférieure/supérieure et qui sont destinées à venir en prise avec la paroi osseuse de la glène G.

Avantageusement, la face interne 12 est recouverte au moins en partie d'une couche d'hydroxyapatite de calcium destinée à faciliter l'ostéo-intégration une fois le composant glénoïdien 2 implanté.

Le corps 12 présente également une face externe 20 portant une surface concave S₂ adaptée pour coopérer de façon articulaire avec la surface convexe S₄ de la tête humérale 8. De façon connue en soi, les géométries respectives de ces surfaces S₂ et S₄ sont conçues pour reproduire aussi fidèlement que possible les comportements articulaires anatomiques de l'épaule. De plus, la courbure de la surface S₂ est adaptée pour limiter autant que possible les risques de luxation de la tête humérale 8 lors des sollicitations courantes de la prothèse 1.

La surface concave S₂ se prolonge à sa périphérie par une surface convexe 22 qui forme l'amorce du chant 11 du côté externe du composant glénoïdien. En d'autres termes, la partie interne du chant 11, sensiblement plane dans le mode de réalisation des figures 1 à 3, et la surface S₂ de la face externe 20 sont raccordées de façon continue par la surface incurvée 22. La forme émoussée de cette surface 22 limite les risques de coupure, d'entaille ou de marquage de la tête 8 lors de la sollicitation de la prothèse 1. A titre d'exemple, le rayon de courbure minimal de la surface 22 est de l'ordre de 1 à 3 mm pour un composant glénoïdien destiné à un adulte.

Pour faciliter l'obtention de la surface émoussée 22, cette dernière se raccorde de préférence tangentiellement à la surface d'articulation S₂, évitant ainsi également tout risque d'entaille de la tête humérale 8.

Lorsque la prothèse totale 1 est implantée et sollicitée, la tête humérale 8 s'appuie sur la face externe 20 du corps glénoïdien 10, les surfaces S₂ et S₄ s'articulant l'une sur l'autre.

Par rapport aux prothèses de l'art antérieur dans lesquelles l'usure est essentiellement supportée par un composant glénoïdien en polyéthylène, la prothèse selon l'invention présente l'avantage de reporter cette usure sur la tête humérale 8, c'est-à-dire sur la surface S₄ d'aire de contact plus grande que celle de la surface S₂. L'usure correspondante s'en trouve ralentie. De plus, l'épaisseur de polyéthylène portant la surface S₄ peut être prévue plus grande que celle envisageable pour un composant glénoïdien de l'art antérieur, le volume utile disponible au niveau de la tête humérale étant supérieur à celui généralement disponible pour le composant glénoïdien, sauf à former une prothèse dont le comportement serait très éloigné du comportement anatomique. On augmente ainsi la durée de vie de la prothèse 1.

Lorsque la prothèse 1 a atteint sa durée de vie prédéterminée, la pièce d'usure formée par la tête humérale 8 est changée, ce qui est plus facile et plus rapide que de changer le composant glénoïdien comme pour les prothèses d'épaule actuelles.

En outre, le corps glénoïdien 10 étant constitué de métal, il est fortement rigide et est plus facilement ancré dans la glène G. Outre les moyens de solidarisation décrits ci-dessus, le corps 10 est, selon une variante non représentée, percé d'orifices traversants débouchant sur la surface d'articulation S₂ et adaptés pour recevoir des vis d'ancrage dans l'os, notamment des vis auto-bloquantes. Avec un composant glénoidien actuel dont le corps est réalisé en un matériau plus mou, tel que le polyéthylène, l'utilisation de telles vis auto-bloquantes est inenvisageable. Ainsi, dans la mesure où le composant glénoïdien selon l'invention permet une fixation osseuse plus efficace, sa pose est envisageable avec ou sans ciment.

Sur les figures 4 et 5 est représentée une variante du composant glénoïdien 2 qui se distingue essentiellement du mode de réalisation des figures précédentes au niveau de son côté interne. En effet, la face interne 12, dépourvue d'aile saillante, est destinée à être cimentée sur la glène G. A cet effet, la face 12 porte dans sa partie centrale une surface 30 adapté pour faciliter la prise du ciment. Cette surface est par exemple microbillée, sablée ou polie.

Pour fixer solidement le composant 2 à la glène, le corps 10 est muni d'une patte antérieure 32 et d'une griffe inférieure 34. Ces éléments 32 et 34 se présentent sous la forme de languettes métalliques, venues de matière avec le corps 10, qui s'étendent en saillie du corps, vers la glène et qui sont reliées au corps au niveau de son chant 11. Plus précisément, la patte 32 et la griffe 34 se raccordent tangentiellement à des parties périphériques correspondantes de la surface émoussée 22.

La patte 32 s'étend suffisamment le long de l'os glénoïdien G pour recevoir une vis d'ancrage osseux 36 représentée sur la figure 5. Cette vis est de préférence autobloquante vis-à-vis de la patte et s'étend avantageusement suivant un angle de rétroversion permettant une reconstruction osseuse de la glène par implantation d'une greffe G' dans la partie postérieure de l'os.

La griffe 34 permet quant à elle de former un appui inférieur pour la glène G.

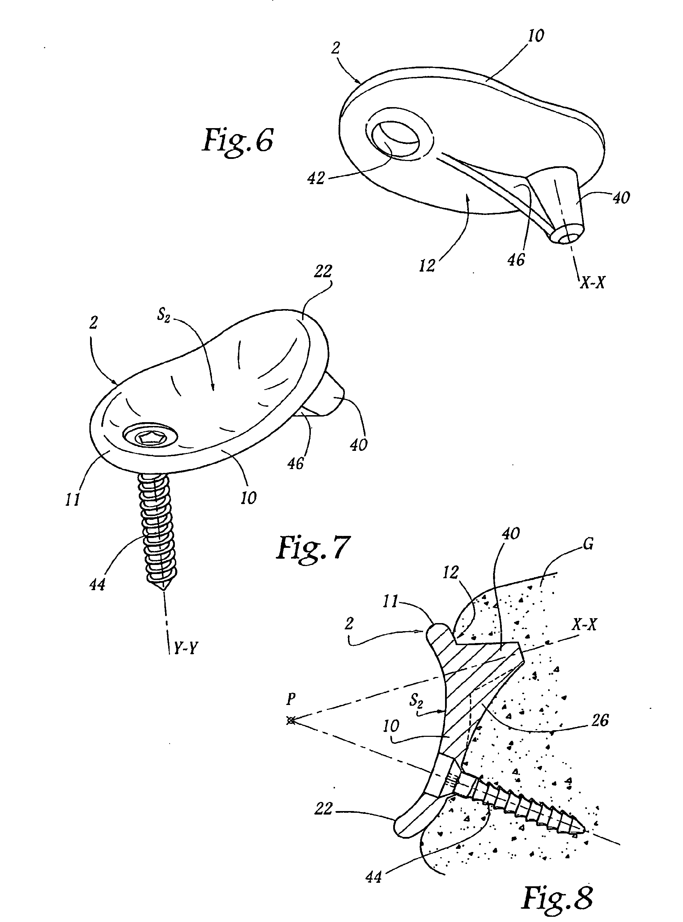

Sur les figures 6 à 8 est représentée une autre variante du composant glénoïdien 2 qui se distingue essentiellement du mode de réalisation des figures 1 à 3 au niveau de son côté interne. En effet, l'aile saillante 14 est remplacée par un plot 40 d'ancrage osseux dans la glène G. Ce plot se présente sous la forme d'un pion globalement tronconique d'axe X-X, qui s'étend en saillie de la face interne 12 du corps 10, en étant venu de matière avec le corps. La surface du plot 40 est divergente en direction de la face 12 de manière à faciliter son introduction dans l'os, éventuellement creusé au préalable de manière sensiblement correspondante.

Le plot 40 s'étend de la partie supérieure de la face interne 12 tandis que la partie inférieure présente un alésage traversant 42 adapté pour recevoir une vis 44 d'ancrage osseux dans la partie basse de la glène G, cette vis étant analogue à la vis 36 de la figure 5. Cette vis 44 est de préférence autobloquante vis-à-vis de l'alésage 42.

Lorsque la vis 44 est reçue dans l'alésage 42, elle s'étend en longueur autour d'un axe central Y-Y qui définit, avec l'axe X-X, un plan sensiblement vertical correspondant au plan de la figure 8. Dans ce plan, les axes X-X et Y-Y sont inclinés l'un par rapport et s'intersectent en un point P situé du côté externe du corps 10, c'est-à-dire du côté du centre de courbure de la surface S₂. Autrement dit les axes X-X et Y-Y divergent l'un de l'autre du côté interne du corps 10 ce qui assure un ancrage fiable, les parties à la fois haute et basse de la glène G étant sollicitées.

Pour limiter les risques de basculement du composant 2 autour d'une direction globalement verticale, une nervure 46 est venue de matière avec la face 12 du corps 10 et s'étant dans le plan de la figure 8, du plot 40 jusqu'au pourtour de l'alésage 42, avec une épaisseur continûment décroissante.

Selon une variante non-représentée, le plot 40 peut être alésé intérieurement suivant son axe X-X, de manière à pouvoir recevoir une autre vis d'ancrage osseux.

D'autres variantes et aménagements au composant glénoïdien et à la prothèse totale décrits ci-dessus sont en outre envisageables. En particulier, les moyens de solidarisation avec la glène ou l'humérus envisagés ci-dessus ne sont nullement limitatifs et peuvent être aménagés par l'homme du métier. De même, la ou les surfaces d'appui 18 ou 30 du corps glénoïdien 10 peuvent être sensiblement planes comme pour le composant glénoïdien des figures 6 à 8, selon l'état osseux de la glène et la géométrie de la résection réalisée pour la pose du composant 2. La glène 2 peut être réalisée en plusieurs parties métalliques solidarisées les unes ou autres.

Par ailleurs, dans la mesure où le corps glénoïdien 10 est en métal, il est facile de prévoir que ce corps s'étende jusqu'à la partie acromiale de l'omoplate du patient, de façon à augmenter la solidarisation du composant glénoïdien sur l'os de l'épaule.

## Revendications

1. Composant glénoïdien (2) de prothèse d'épaule (1) avec une surface d'articulation concave (S₂), laquelle surface d'articulation se prolonge en périphérie par une surface convexe (22) formant au moins en partie le chant (11) de ce composant glénoïdien, **caractérisé en ce que** le composant glénoïdien comprend un corps métallique (10) adapté, au niveau d'une (12) de ses faces, pour être immobilisé sur la glène (G) d'une épaule, et présentant, sur une face opposée (20), la surface concave d'articulation (S₂), adaptée pour coopérer avec un composant huméral (4) de la prothèse (1), et la surface convexe (22).

2. Composant suivant la revendication 1, **caractérisé en ce que** ladite surface convexe (22) et la surface d'articulation (S₂) se raccordent tangentiellement l'une à l'autre.

3. Composant suivant l'une des revendications 1 ou 2, **caractérisé en ce que** le rayon de courbure minimal de ladite surface convexe (22) est compris entre environ 1 et 3 mm.

4. Composant suivant l'une quelconque des revendications précédentes, **caractérisé en ce que** le corps métallique (10) est d'un seul tenant.

5. Composant suivant l'une quelconque des revendications précédentes, **caractérisé en ce que** le corps métallique (10) est pourvu d'au moins un orifice traversant (42) débouchant sur la surface d'articulation (S₂) et adaptés pour recevoir des moyens (44) d'ancrage du composant (2) dans l'os de l'épaule, notamment des moyens d'ancrage auto-bloquants.

6. Composant suivant l'une quelconque des revendications précédentes, **caractérisé en ce que** le corps métallique (10) est muni, du côté destiné à être immobilisé sur la glène (G), d'au moins deux éléments (40, 44) d'ancrage osseux définissant des directions respectives (X-X, Y-Y) d'application dans l'os de la glène, divergentes l'une de l'autre en s'éloignant du corps métallique.

7. Composant suivant l'une quelconque des revendications précédentes, **caractérisé en ce que** le corps métallique (10) est muni, du côté (12) destiné à être immobilisé sur la glène (G), d'au moins une languette saillante (32, 34) qui s'étend dans le prolongement d'une partie de ladite surface convexe (22).

8. Composant suivant la revendication 7, **caractérisé en ce que** la ou les languettes (32, 34) sont venues de matière avec le corps (10).

9. Composant suivant l'une des revendications 7 ou 8, **caractérisé en ce que** la ou une des languettes forme, de préférence dans la partie antérieure du composant glénoidien (2), une patte (32) de fixation osseuse adaptée pour recevoir une vis (36) d'ancrage osseux.

10. Composant suivant l'une quelconque des revendications 7 à 9, **caractérisé en ce que** la ou une des languettes forme, de préférence dans la partie inférieure du composant glénoidien (2), une griffe (34) d'appui pour la glène (G).

11. Composant suivant l'une quelconque des revendications précédentes, **caractérisé en ce que** la face (12) du corps métallique (10) adaptée pour être immobilisée sur la glène (G) est recouverte d'une couche active pour faciliter l'ostéo-intégration, notamment d'une couche en hydroxyapatite de calcium.

12. Composant suivant l'une quelconque des revendications 1 à 10, **caractérisé en ce que** la face (12) du corps métallique (10) adaptée pour être immobilisée sur la glène (G) présente un état de surface prévu pour faciliter le cimentage du composant glénoïdien (2).

13. Prothèse totale d'épaule (1), **caractérisée en ce qu'**elle comporte un composant glénoïdien (2) suivant l'une quelconque des revendications précédentes et un composant huméral (4) portant une surface convexe (S₄) de coopération articulaire avec la surface concave (S₂) du composant glénoïdien.

14. Prothèse suivant la revendication 13, **caractérisée en ce que** la surface convexe (S₄) du composant huméral (4) est portée par une pièce (8) de ce composant constituée d'un matériau plus mou que le métal du corps (10) du composant glénoïdien (2), notamment une matière plastique.

## Claims

1. Glenoidal component (2) of a shoulder prosthesis (1) with a concave articulation surface (S₂), said articulation surface being extended at the periphery by a convex surface (22) forming at least in part the sharp edge (11) of this glenoidal component, **characterised in that** the glenoidal component comprises a metallic body (10) adapted on one of its faces (12) so as to be immobilised on the glene (G) of a shoulder, and having on an opposed face (20) the concave articulation surface (S₂), adapted so as to interact with a humeral component (4) of the prosthesis (1), and the convex surface (22).

2. Component according to Claim 1, **characterised in that** the said convex surface (22) and the articulation surface (S₂) are connected tangentially to one another.

3. Component according to either of Claims 1 and 2, **characterised in that** the minimum curvature radius of the said convex surface (22) is comprised between about 1 and 3 mm.

4. Component according to any one of the preceding claims, **characterised in that** the metallic body (10) is of one single piece.

5. Component according to any one of the preceding claims, **characterised in that** the metallic body (10) is provided with at least one transverse aperture (42) opening onto the articulation surface (S₂) and adapted to accommodate means (44) for anchoring the component (2) in the bone of the shoulder, in particular by means of self-blocking anchoring means.

6. Component according to any one of the preceding claims, **characterised in that** the metallic body (10) is provided on the side intended to be immobilised on the glene (G) with at least two bone anchoring elements (40, 44), defining the respective directions (X-X, Y-Y) of application in the bone of the glene, diverging from one another as they become more distant from the metallic body.

7. Component according to any one of the preceding claims, **characterised in that** the metallic body (10) is provided on the side (12) intended to be immobilised on the glene (G) with at least one projecting tongue (32, 34) which extends into the extension of a part of the said convex surface (22).

8. Component according to Claim 7, **characterised in that** the tongue (s) (32, 34) are made of the same material as the body (10).

9. Component according to either of Claims 7 and 8, **characterised in that** the tongue(s) form(s), preferably in the front part of the glenoidal component (2), a bone fixation lug (32) adapted so as to receive a bone anchoring screw (36).

10. Component according to any one of the preceding Claims 7 to 9, **characterised in that** the tongue(s) form(s), preferably in the lower part of the glenoidal component (2), a support grip element (34) for the glene (G).

11. Component according to any of the preceding claims, **characterised in that** the face (12) of the metallic body (10) adapted so as to be immobilised on the glene (G) is covered with an active layer so as to facilitate osteointegration, in particular a layer of calcium hydroxyapatite.

12. Component according to any one of the preceding Claims 1 to 10, **characterised in that** the face (12) of the metallic body (10) adapted so as to be immobilised on the glene (G) has a surface condition provided so as to facilitate the cementing of the glenoidal component (2).

13. Total shoulder prosthesis (1), **characterised in that** it comprises a glenoidal component (2) according to any one of the preceding claims and a humeral component (4) having a convex surface (S₄) for articular interaction with the concave surface (S₂) of the glenoidal component.

14. Prosthesis according to Claim 13, **characterised in that** the convex surface (S₄) of the humeral component (4) is carried by a piece (8) of this component, composed of a softer material than the metal of the body (10) of the glenoidal component (2), in particular a plastic material.

## Patentansprüche

1. Glenoidkomponente (2) einer Schulterprothese (1) mit einer konkaven Artikulationsoberfläche (S₂), wobei sich die Artikulationsoberfläche an der Peripherie in einer konvexen Oberfläche (22) fortsetzt, die zumindest teilweise die Kante (11) der Glenoidkomponente bildet, **dadurch gekennzeichnet, dass** die Glenoidkomponente einen metallischen Körper (10) umfasst, der auf einer seiner Flächen (12) dafür ausgelegt ist, an der Gelenkpfanne (G) einer Schulter immobilisiert zu werden, und der auf seiner gegenüberliegenden Seite (20) die konkave Artikulationsoberfläche (S₂), die dafür ausgelegt ist, mit einer Humeruskomponente (4) der Prothese (1) zusammenzuwirken, und die konvexe Oberfläche (22) aufweist.

2. Komponente nach Anspruch 1, **dadurch gekennzeichnet, dass** die konvexe Oberfläche (22) und die Artikulationsoberfläche (S₂) tangential aneinander stoßen.

3. Komponente nach einem der Ansprüche 1 oder 2, **dadurch gekennzeichnet, dass** der minimale Krümmungs-radius der konvexen Oberfläche (22) zwischen etwa 1 und 3 mm beträgt.

4. Komponente nach einem der vorhergehenden Ansprüche, **dadurch gekennzeichnet, dass** der metallische Körper (10) zusammenhängend ist.

5. Komponente nach einem der vorhergehenden Ansprüche, **dadurch gekennzeichnet, dass** der metallische Körper (10) mit mindestens einer hindurch gehenden Öffnung (42) versehen ist, die auf der Artikulations-oberfläche (S₂) mündet und dafür ausgelegt ist, Mittel (44) zur Verankerung der Komponente (2) im Schulter-knochen, insbesondere selbstblockierende Verankerungsmittel, aufzunehmen.

6. Komponente nach einem der vorhergehenden Ansprüche, **dadurch gekennzeichnet, dass** der metallische Körper (10) auf der Seite, die für die Immobilisierung an der Gelenkpfanne (G) bestimmt ist, mit mindestens zwei Knochenverankerungselementen (40, 44) ausgestattet ist, welche die jeweiligen Richtungen (X-X, Y-Y) des Ansetzens der Gelenkpfanne im Knochen definieren, die bei der Entfernung vom metallischen Körper voneinander divergieren.

7. Komponente nach einem der vorhergehenden Ansprüche, **dadurch gekennzeichnet, dass** der metallische Körper (10) auf der Seite (12), die für die Immobilisierung an der Gelenkpfanne (G) bestimmt ist, mit mindestens einer vorspringenden Lasche (32, 34) ausgestattet ist, die sich in der Verlängerung eines Teils der konvexen Oberfläche (22) erstreckt.

8. Komponente nach Anspruch 7, **dadurch gekennzeichnet, dass** die Lasche(n) (32, 34) mit dem Körper (10) stofflich verbunden ist (sind).

9. Komponente nach einem der Ansprüche 7 oder 8, **dadurch gekennzeichnet, dass** die Lasche oder eine der Laschen vorzugsweise im anterioren Teil der Glenoidkomponente (2) eine Knochenbefestigungslasche (32) bildet, die dafür ausgelegt ist, eine Knochenverankerungsschraube (36) aufzunehmen.

10. Komponente nach einem der Ansprüche 7 bis 9, **dadurch gekennzeichnet, dass** die Lasche oder eine der Laschen vorzugsweise im unteren Teil der Glenoidkomponente (2) eine Auflageklammer (34) für die Gelenkpfanne (G) bildet.

11. Komponente nach einem der vorhergehenden Ansprüche, **dadurch gekennzeichnet, dass** die Fläche (12) des metallischen Körpers (10), die für die Immobilisierung an der Gelenkpfanne (G) bestimmt ist, mit einer aktiven Schicht vor Ermöglichung der Osteointegration, insbesondere mit einer Calciumhydroxylapatit-Schicht bedeckt ist.

12. Komponente nach einem der Ansprüche 1 bis 10, **dadurch gekennzeichnet, dass** die Fläche (12) des metallischen Körpers (10), die für die Immobilisierung an der Gelenkpfanne (G) bestimmt ist, einen Oberflächenzustand aufweist, um die Zementierung der Glenoidkomponente (2) zu ermöglichen.

13. Schultertotalprothese (1), **dadurch gekennzeichnet, dass** sie eine Glenoidkomponente (2) nach einem der vorhergehenden Ansprüche und eine Humeruskomponente (4) enthält, die eine konvexe Oberfläche (S₄) für das gelenkige Zusammenwirken mit der konkaven Oberfläche (S₂) der Glenoidkomponente aufweist.

14. Prothese nach Anspruch 13, **dadurch gekennzeichnet, dass** die konvexe Oberfläche (S₄) der Humeruskomponente (4) sich auf einem Stück (8) dieser Komponente befindet, die aus einem weicheren Material als das Metall des Körpers (10) der Glenoidkomponente (2), insbesondere aus einem Kunststoffmaterial, besteht.
